# EUROPÄISCHE PATENTSCHRIFT

(11) **EP 2 035 118 B1**
(45) Veröffentlichungstag und Bekanntmachung des Hinweises auf die Patenterteilung: **12.04.2017**
(21) Anmeldenummer: 07730182.8
(22) Anmeldetag: 15.06.2007
(51) Int. Cl.: B01D 53/62, B01D 53/86, B01J 20/06, B01J 20/30, B01J 21/06, B01J 23/72, B01D 53/02, B01J 37/03, B01J 20/34, B01J 37/00, C10K 1/32, C10K 3/04, C07C 7/12, B01J 20/32

(54) **VERFAHREN ZUR ENTFERNUNG VON CO AUS STOFFSTRÖMEN**
METHOD FOR ELIMINATING CO FROM STREAMS OF SUBSTANCES
PROCÉDÉ PERMETTANT D'ÉLIMINER DU CO DE FLUX DE MATIÈRE

(30) Priorität: 21.06.2006 EP 06115823
(43) Veröffentlichungstag der Anmeldung: 18.03.2009
(73) Patentinhaber: BASF SE, 67056 Ludwigshafen am Rhein (DE)
(72) Erfinder: HATSCHER, Stephan, 28857 Syke (DE); HESSE, Michael, 67549 Worms (DE)
(74) Vertreter: BASF IP Association
(86) Internationale Anmeldenummer: PCT/EP2007/055938
(87) Internationale Veröffentlichungsnummer: WO 2007/147783

(56) Entgegenhaltungen:
- EP-A- 0 691 157
- WO-A-2004/080589
- WO-A-2007/093526
- DE-A1- 4 021 230
- DE-A1- 10 241 529
- DE-A1-102005 061 322
- JUNG BONG KO ET AL: "Cu-ZrO2 Catalysts for Water-gas-shift Reaction at Low Temperatures" CATALYSIS LETTERS, KLUWER ACADEMIC PUBLISHERS-PLENUM PUBLISHERS, NE, Bd. 105, Nr. 3-4, 1. Dezember 2005 (2005-12-01), Seiten 157-161, XP019275312 ISSN: 1572-879X
- WEI LU AND MARIA FLYTZANI-STEPHANOPOULOS: "Total Oxidation of Carbon Monoxide and methane over Transition Metal-Fluorite Oxide Composite Catalysts" JOURNAL OF CATALYSIS, Bd. 153, 1995, Seiten 304-316, XP002470212

## Beschreibung

Die vorliegende Erfindung betrifft ein Verfahren zur Entfernung von Kohlenmonoxid (CO) aus flüssigen Propylenstrom gemäß Anspruch 1.

In verschiedenen Gebieten der Technik ist es wichtig, besonders reine Stoffströme zur Verfügung zu haben. "Rein" bedeutet in diesem Zusammenhang, dass der Stoffstrom frei von Bestandteilen ist, die bei der bestimmungsgemäßen Verwendung des Stoffstroms störend wirken. Ein Beispiel ist Atemluft, die frei von toxischen Verbindungen sein muss. Ebenso werden etwa bei der Herstellung von elektronischen Bauteilen reine Stoffströme benötigt, um keine Kontaminationen einzuschleppen, die die elektronischen Eigenschaften der hergestellten Bauteile beeinträchtigen, unter anderem wird dabei oft besonders reiner Stickstoff oder besonders reines Argon als Schutzgas benötigt. Ein anderes Beispiel sind katalytische chemische Reaktionen. Katalysatoren sind oft sehr empfindlich gegen Vergiftungen. Da man aus wirtschaftlichen Gründen üblicherweise den pro Volumen oder Masse des Katalysators einzusetzenden Einsatzstoffstrom zu maximieren sucht, können schon außerordentlich kleine Mengen an Verunreinigungen im Einsatzstoffstrom sich auf dem Katalysator ansammeln und diesen vergiften. Typischerweise werden für Olefinpolymerisationsreaktionen an modernen Katalysatoren - beispielsweise Metallocenkatalysatoren - Olefinströme benötigt, die nicht mehr als einige ppb (parts per billion, d.h., 10⁻⁹ Anteile Verunreinigungen pro Anteil des gewünschten Stoffs) enthalten ("polymer grade"-Olefine). Aus typischen Olefinquellen (Steamcracker, Fluid Catalytic Cracker, Dehydrierungen, MTO-Verfahren ("Methanol to Olefins") stammende Olefine enthalten meist sehr viel höhere Anteile (ppm-oder sogar Promille-Bereich) an Verunreinigungen wie Kohlenmonoxid oder Sauerstoff ("chemical grade"); diese Anteile müssen vor der Verwendung zur Polymerisation entsprechend erniedrigt werden.

Typischerweise sind die zu reinigenden Stoffströme Luft, Stickstoff oder Argon oder Kohlenwasserstoffe wie Ethylen, Propylen, 1-Buten, 2-Buten, 1,3-Butadien oder Styrol. Typische Verunreinigungen, die in aller Regel entfernt werden müssen, sind Sauerstoff und Kohlenmonoxid, und oft auch Wasser, Kohlendioxid, Wasserstoff, oder auch Schwefel-, Arsen- oder Antimonverbindungen. Verfahren zur Entfernung solcher Verunreinigungen aus Stoffströmen sind bekannt.
Am bekanntesten ist die Entfernung von Kohlenmonoxid aus sauerstoffhaltigen Gasströmen, beispielsweise aus Atemluft. Dies geschieht meist durch katalytische Umsetzung von Kohlenmonoxid mit Sauerstoff, in der Regel an Kupfer enthaltenden Katalysatoren. Der meistverwendete Katalysator dieser Reaktion ist Hopcalit, ein ursprünglich für die CO-Entfernung aus Atemluft in Atemmasken entwickeltes, für die Umsetzung von Kohlenmonoxid mit Sauerstoff höchst aktives Kupfer-Mangan-Mischoxid, an dem sich das hochtoxische Kohlenmonoxid mit Sauerstoff zu Kohlendioxid umsetzt.

Es sind jedoch auch andere Verwendungen von Hopcalit und Verfahren zur Reinigung anderer Stoffströme als Atemluft bekannt. So offenbart WO 98/041 597 A1 ein Verfahren zur Entfernung von Alkinen, einfach oder mehrfach ungesättigten Kohlenwasserstoffen, Schwefel-, Antimon- oder Arsenverbindungen, Sauerstoff, Wasserstoff und Kohlenmonoxid aus Stoffströmen durch eine Abfolge von zwei oder drei bestimmten katalytischen und absorptiven Verfahrensschritten. EP 662 595 A1 lehrt ein Verfahren zur Entfernung von Wasserstoff, Kohlenmonoxid und Sauerstoff aus kaltem flüssigem Stickstoff durch Inkontaktbringen mit bestimmten Zeolithen oder anderen Metalloxiden, insbesondere Hopcalit. EP 750 933 A1 offenbart ein ähnliches Verfahren zur Entfernung von Sauerstoff und Kohlenmonoxid aus kaltem Stickstoff oder kalten Edelgasen durch Inkontaktbringen mit Metalloxiden, insbesondere Hopcalit. Bei den angewendeten tiefen Temperaturen unterhalb von -40 °C findet allerdings keine oder nur wenig katalytische Reaktion statt, Sauerstoff und Kohlenmonoxid werden am Hopcalit adsorbiert und reagieren erst bei höherer Temperatur ab, es sei denn, sie werden in der Kälte in einem Desorptionsschritt entfernt. EP 820 960 A1 offenbart ein Verfahren zur Entfernung von Sauerstoff und Kohlenmonoxid aus Stickstoff oder Edelgasen durch Inkontaktbringen mit Metalloxiden wie Hopcalit, insbesondere bei Temperaturen von 5 bis 50°C.

T.-J Huang und D.-H. Tsai, Catalysis Letters 87 (2003) 173-178 berichten über Untersuchungen zum Einfluss des Oxidationsgrads des Kupfers auf die Oxidation von Kohlenmonoxid. Cu₂O ist insgesamt aktiver als CuO, was auf die höhere Beweglichkeit von Sauerstoff in Cu₂O, verglichen mit Cu oder CuO zurückgeführt wird.

WO 02/094 435 A1 lehrt ein Verfahren zum oxidativen Entfernen von CO aus Ethylen bei Temperaturen im Bereich von 70 bis 110 °C an Kupfer und Zink enthaltenden Katalysatoren.

WO 02/026 619 A2 offenbart ein Verfahren zur Entfernung von Kohlenmonoxid durch Wassergas-Shift-Reaktion und WO 03/051 493 A2 ein Verfahren zur selektiven Oxidation von Kohlenmonoxid, jeweils in Gasströmen, die Kohlenmonoxid, Sauerstoff und Wasserstoff enthalten, insbesondere in Brennstoffzellen, und jeweils an Katalysatoren, die Kupfer, ein Metall der Platingruppe und ein reduzierbares Metalloxid auf einem oxidischen Träger aus aktiviertem Aluminium, Zirkondioxid, Titandioxid, Siliciumdioxid, Zeolithen oder ihre Kombinationen enthalten. Das reduzierbare Metalloxid wird aus der Gruppe der Oxide von Cr, V, Mo, Ce, Pr, Nd, Ti, Ni, Mn, Co und ihren Kombinationen gewählt. US 6 238 640 B1 beschreibt ein Verfahren zur Entfernung von Kohlenmonoxid aus Wasserstoff enthaltenden Gasströmen durch Umsetzung mit Dampf und Sauerstoff zu Kohlendioxid und Wasserstoff in Gegenwart eines Katalysators, der Kupfer- und Aluminiumoxid sowie mindestens ein Metalloxid aus der von Zinkoxid, Chromoxid und Magnesiumoxid gebildeten Gruppe enthält.

Bei diesen Verfahren zur Entfernung von Kohlenmonoxid in Gegenwart von Sauerstoff durch deren Umsetzung entsteht Kohlendioxid. Dies kann in nachfolgenden Verfahren inert sein oder selbst eine störende Verunreinigung darstellen. Im letzteren Fall wird es entfernt, auch hierfür sind verschiedene Verfahren bekannt. Beispielsweise lehrt CA 2 045 060 A1 ein Verfahren zur Entfernung von Kohlenmonoxid, Kohlendioxid, Wasserstoff, Sauerstoff und Wasserdampf aus Inertgasströmen bei einer Temperatur im Bereich von -30°C bis + 40°C, insbesondere von -30°C und 0 °C, wobei Kohlenmonoxid an Übergangsmetalloxiden wie Hopcalit oder Kupfer-Kobalt-Oxid zu Kohlendioxid umgesetzt wird und letzteres durch Adsorption an Kupfer auf einem Aluminiumoxidträger oder Nickel auf einem Aluminiumoxid- oder Siliciumdioxid-Träger entfernt wird.

Bei manchen Anwendungen muss Kohlenmonoxid jedoch auf andere Weise als durch Umsetzung mit Sauerstoff oder Wasser entfernt werden, beispielsweise dann, wenn zwar Kohlenmonoxid, aber kein Sauerstoff, kein Wasser oder lediglich ein stöchiometrischer Unterschuss davon im zu reinigenden Stoffstrom enthalten ist. In manchen Anwendungsfällen muss Sauerstoff vor dem Kohlenmonoxid entfernt werden, insbesondere dann, wenn neben der Bildung von Kohlendioxid auch sonstige störende Nebenprodukte gebildet werden können. Beispielsweise können bei der Entfernung von Sauerstoff und Kohlenmonoxid an Kupfer enthaltenden Katalysatoren aus flüssigen Kohlenwasserstoffen wie Propylen, Buten, Butadien oder Styrol auch Oxidationsprodukte des Kohlenwasserstoffs gebildet werden (sogenannte "Oxygenate"), die selbst störende Verunreinigungen darstellen. In solchen Fällen muss der Sauerstoff vor der Entfernung des Kohlenmonoxids entfernt werden, und Kohlenmonoxid kann nicht durch Oxidation entfernt werden.

In solchen Fällen wird Kohlenmonoxid daher üblicherweise durch Destillation entfernt, damit ist jedoch keine CO-Entfernung bis auf Restgehalte im ppb-Bereich möglich. Es sind aber auch adsorptive Verfahren und Adsorbentien zur Reinigung von Stoffströmen bekannt. Die deutsche Offenlegungsschrift DE 1 929 977 lehrt 20 bis 60 Teile CuO auf 100 Teile ZnO enthaltende Katalysatoren und ihre Verwendung zur Entfernung von CO aus Ethylen- und Propylenströmen bei einer Temperatur im Bereich von 50 bis 200°C. US 3 676 516 lehrt einen geträgerten Cu-Katalysator, dessen Kupfer zu 20 bis 95 % als Cu²⁺ vorliegt, und seine Verwendung zur CO-Entfernung aus Ethylen- oder Propylenströmen bei einer Temperatur unterhalb von rund 200 °C, in den Beispielen konkret bei rund 93 °C. US 4 917 711 offenbart ein Adsorbens, das eine Kupferverbindung auf einem hochoberflächigen Träger enthält, jedoch auch Olefine adsorbiert und daher nur zur Reinigung von Stickstoff, Edelgasen und gesättigten Kohlenwasserstoffen geeignet ist. WO 01/007 383 A1 lehrt ein Verfahren zur Reinigung von Olefinströmen durch Überleiten über poröse Adsorbentien wie Ruß oder Aluminium- und/oder Siliciumoxide. JP 02 144 125 A2 (CAS Abstract 113:177 506) lehrt ein Verfahren zur Entfernung von Kohlenmonoxid und Metallcarbonylen aus bei der Halbleiterfertigung entstehenden Abgasen durch Adsorption an Manganoxid und Kupferoxid enthaltenden Adsorptionsmassen. JP 05 337 363 A2 (CAS Abstract 120:274 461) offenbart Adsorbentien zur Kohlenmonoxidentfernung, die Palladium und weitere Oxide auf einem Träger enthalten, wobei die Oxide aus den Oxiden der Elemente der Gruppen 11, 2 und 12 (ohne Be, Cd, Hg und Ra), 13 (ohne Al, TI und den Actiniden), 14 (ohne C, Si, Pb und Hf), 5 und 15 (ohne N, P, As und die "Pa-Reihe"), 6 und 16 (ohne O, S, Se und U), 7 und 8 des Periodensystems der Elemente gewählt werden.

WO 95/021 146 A1 lehrt ein Verfahren zur Entfernung von Kohlenmonoxid und, falls vorhanden, auch Arsin aus flüssigen Kohlenwasserstoffströmen durch Inkontaktbringen mit einem Sorbens, das je nach Ausführungsform disperses Kupfer in den Oxidationstufen 0, +1 oder +2, und in bestimmten Fällen auch Mangandioxid enthält.
EP 537 628 A1 offenbart ein Verfahren zur Entfernung von Kohlenmonoxid aus alpha-Olefinen und gesättigten Kohlenwasserstoffen durch Inkontaktbringen mit einem Katalysatorsystem auf Basis mindestens eines Oxids eines aus Cu, Fe, Ni, Co, Pt und Pd gewählten Metalls und mindestens eines Oxids eines aus den Gruppen 5, 6 oder 7 des Periodensystems der Elemente gewählten Metalls bei 0 bis 150 °C. US 4713 090 beschreibt ein Adsorbens zur Gewinnung hochreinen Kohlenmonoxids durch Druck- oder Temperaturwechseladsorption. Das Adsorbens umfasst einen zusammengesetzten Träger mit einem Kern aus Silicium- oder Aluminiumoxid und einer Außenschicht aus einer Aktivkohle, auf der eine Kupferverbindung geträgert ist.

WO 2004/022 223 A2 lehrt eine Kupfer, Zink, Zirkon und wahlweise Aluminium enthaltende Adsorptionsmasse und ihre Verwendung zur Entfernung von CO aus Stoffströmen in vollständig reduziertem Zustand.

Kupferhaltige Katalysatoren sind auch zu anderen Anwendungszwecken als zur Entfernung von CO aus Inertgasen oder Kohlenwasserstoffen bekannt. US 4 593 148 und US 4 871 710 offenbaren zur Entschwefelung und Entarsenierung Cu/Zn-Katalysatoren. WO 95/023 644 A1 lehrt einen Kupferkatalysator zur Hydrierung von Kohlenoxiden, beispielsweise zu Methanol, oder für die sogenannte Shift-Reaktion von Kohlenmonoxid mit Wasser zu Kohlendioxid und Wasserstoff, der neben dispersem Kupfer auch Stabilisatoren wie Siliciumdioxid, Aluminiumoxid, Chromoxid, Magnesiumoxid und/oder Zinkoxid und wahlweise auch einen Träger wie Aluminiumoxid, Zirkondioxid, Magnesiumoxid und/oder Siliciumdioxid enthält, und seine Aktivierung und Passivierung. DE 198 48 595 A1 offenbart einen Katalysator zur Lachgaszersetzung der allgemeinen Formel MₓAl₂O₄, in denen M Cu oder eine Mischung aus Cu und Zn und/oder Mg ist und der weitere Dotierungen, insbesondere Zr und/oder La enthalten kann. US 5 328 672 lehrt einen Autoabgasreinigungskatalysator, der aus einem ein

Übergangsmetall enthaltenden Oxid und einem ein Übergangsmetall enthaltenden Zeolith besteht, wobei das Übergangsmetall aus Cu, Co, Ni, Cr, Fe, Mn, Ag, Zn, Ca und "kompatiblen Mischungen davon" gewählt wird, vorzugsweise in Oxid und Zeolith identisch und besonders bevorzugt Cu ist und das Oxid aus La-, Ti-, Si-, Zr-Oxid gewählt wird und vorzugsweise ZrO₂ ist. EP 804 959 A1 offenbart einen NOx-Zersetzungskatalysator, der zusätzlich zu Kupfer und einem MFI-Zeolith auch SiO₂, Al₂O₃, SiO₂/Al₂O₃, MgO, ZrO₂ und Ähnliches sowie beliebige weitere Elemente wie die Übergangselemente Pt, Rh, Cr, Co, Y, Zr, V, Mn, Fe und Zn sowie Ga, In, Sn, Pb, P, Sb, Mg und Ba, bevorzugt P enthalten kann. DE 199 50 325 A1 lehrt einen Spinellmonolithkatalysator zur NOₓ-Zersetzung mit der allgemeinen Formel AₓB(₁₋ₓ)E₂O₄, in der A Cu ist, das bis zur Hälfte durch Co, Fe, Ni, Mn oder Cr ersetzt sein kann; B mindestens ein Element, ausgewählt aus Zn, Mg, Ca, Zr, Ce, Sn, Ti, V, Mo und W ist und E Al ist, das bis zur Hälfte durch Fe, Cr, Ga, La oder deren Gemische ersetzt sein kann. US 4 552 861 lehrt ein Herstellverfahren für Katalysatoren, die Cu, Zn, Al und mindestens ein Element aus der von den Seltenen Erden und Zirkon gebildeten Gruppe enthalten sowie deren Verwendung zur Methanolsynthese. Die in US 4 780 481 offenbarten Methanolkatalysatoren enthalten Cu, Zn und mindestens ein Alkali- oder Erdalkalimetall, Edelmetalle und/oder Seltene Erden, wobei Zn teilweise durch Zr ersetzt werden kann. WO 96/014 280 A1 lehrt Katalysatoren, die Cu, Zn und mindestens eine Verbindung von Al, Zr, Mg, eines Seltenerdmetalls und/oder Mischungen davon enthalten und ihre Verwendung zur Hydrierung von Carbonsäureestern. EP 434 062 A1 lehrt ebenfalls ein Verfahren zur Hydrierung von Carbonsäureestern an einem Katalysator, umfassend Cu, Al und ein aus der von Mg, Zn, Ti, Zr, Sn, Ni, Co und ihren Mischungen gebildeten Gruppe gewähltes Metall. US 4 835 132 beschreibt CO-Shift-Katalysatoren, die aus einem Vorläufer der Formel (Cu+Zn)₆AlₓR_{y}(CO₃)_{(x+y)/2}OH_{12+2(x+y)}nH2O mit Schichtstruktur durch Kalzination erzeugt werden, wobei R La, Ce oder Zr ist, x mindestens 1 und höchstens 4, y mindestens 0,01 und höchstens 1,5 und n ungefähr 4 ist.

Es sind auch Verfahren bekannt, Katalysatoren, auch Kupfer enthaltende, zu aktivieren, zu reaktivieren oder für den Transport zu passivieren. Die Patentschrift DD 0 153 761 betrifft ein Verfahren zur Aktivierung oder Reaktivierung von Eisenmolybdat-Redoxkatalysatoren, die auch Kupfer enthalten können, wobei die Katalysatoren zuerst in nichtoxidierender Atmosphäre kalziniert und dann mit einem oxidierenden Gas in Kontakt gebracht werden. DE 199 63 441 A1 lehrt ein Verfahren zur Regenerierung von Kupfer enthaltenden Hydrierkatalysatoren durch zuerst oxidierende und dann reduzierende Behandlung, wobei die Reduzierung vorzugsweise erst im Hydrierreaktor durchgeführt wird. WO 02/068 119 A1 offenbart kupferhaltige Hydrier- und Dehydrierkatalysatoren, die in reduziertem Zustand eingesetzt und zum Transport durch teilweise Oxidation des Kupfers passiviert werden. EP 296 734 A1 beschreibt Kupfer enthaltende Shift- oder Methanolkatalysatoren, die durch Reduktion bei einer Temperatur unterhalb von 250 °C eine Cu-Oberfläche von mindestens 70 m²/g, bezogen auf Kupfer aufweisen. Derartige Aktivierungs-, Regenerierungs- und Passivierungsverfahren sind auch für andere Katalysatoren bekannt, so offenbart etwa JP 55/003 856 A (WPI-Abstract No. WP198013664C) ein Verfahren zur Aktivierung von Katalysatoren auf Palladiumbasis durch Reduzierung mit Methanol, Oxidation mit Sauerstoff, dann mit Essigsäure und Sauerstoff und abschießende Reduktion mit Wasserstoff.
WO 03/002 252 A1 beschreibt ein Aktivierungsverfahren für einen Kobalt enthaltenden Katalysator durch Behandlung mit Kohlenwasserstoff.
Die steigenden Anforderungen an die Reinheit von Stoffströmen für manche Anwendungsgebiete machen jedoch neue und verbesserte Hilfsmittel und Verfahren zur Entfernung von Verunreinigungen notwendig. Insbesondere problematisch ist die Entfernung von Kohlenmonoxid aus Kohlenwasserstoffen, und dort besonders aus typischerweise in flüssiger Form vorliegenden Kohlenwasserstoffen wie Propen, 1- oder 2-Buten. Dieser Erfindung liegt daher die Aufgabe zu Grunde, ein verbessertes Verfahren zur adsorptiven Entfernung von Kohlenmonoxid aus Stoffströmen zu finden.
Demgemäß wurde eine Kupfer- und Zirkonoxide, jedoch kein Zinkoxid enthaltende Adsorptionsmasse gefunden, die dadurch gekennzeichnet ist, dass sie 30 bis 99,8 Gew.-% Kupferoxid und 0,2 bis 70 Gew.-% Zirkonoxid, bezogen auf die Gesamtmenge der Adsorptionsmasse enthält. Ferner wurden Verfahren zur Entfernung von Kohlenmonoxid aus Stoffströmen gefunden, der Umsetzung von Kohlenmonoxid mit Sauerstoff oder als Reaktionspartner des Kohlenmonoxids gekennzeichnet sind. Weiterhin wurde ein Verfahren zur Entfernung von Kohlenmonoxid aus Stoffströmen durch Adsorption gefunden, das dadurch gekennzeichnet ist, dass man den Kohlenmonoxid enthaltenden Stoffstrom mit einer Adsorptionsmasse in Kontakt bringt, die dadurch gekennzeichnet ist, dass sie 30 bis 99,8 Gew.-% Kupferoxid und 0,2 bis 70 Gew.-% Zirkonoxid, bezogen auf die Gesamtmenge der Adsorptionsmasse, jedoch kein Zinkoxid enthält.

Adsorptionsmasse ist gut geeignet zur Anwendung in Verfahren zur Reinigung von Stoffströmen, insbesondere zur Entfernung von Kohlenmonoxid (CO) aus flüssigen Kohlenwasserstoffen wie Propylen. Ein besonderer Vorteil der erfindungsgemäßen Adsorptionsmasse ist ihre gute Regenerierbarkeit. Die Adsorptionsmasse weist zwar nicht die maximal mögliche Adsorptionskapazität derartiger Zusammensetzungen für CO auf, sie ist jedoch erheblich besser regenerierbar als Zusammensetzungen mit höherer CO-Aufnahmefähigkeit. Sie ist damit hervorragend geeignet, in Anlagen mit zwei Adsorbern, von denen jeweils einer zur Adsorption eingesetzt und einer regeneriert wird, auch Stoffströme mit stark schwankendem CO-Gehalt von CO zu befreien.

Die Adsorptionsmasse wirkt im erfindungsgemäßen adsorptiven Verfahren durch Adsorption. Unter Adsorption wird die Anlagerung eines Adsorbats an die Oberfläche einer Adsorptionsmasse ("Adsorbens") bezeichnet, die im Allgemeinen durch Desorption reversibel ist. Das Adsorbat kann am Adsorbens auch chemisch umgesetzt werden, bleibt das Adsorbens dabei chemisch im wesentlichen unverändert, spricht man von Katalyse (Beispiel: das bekannte Verfahren zur Umsetzung von CO mit Sauerstoff an einem metallischem Kupferkatalysator zu Kohlendioxid), setzt sich das Adsorbat chemisch mit dem Adsorbens um, von Absorption (Beispiele: das bekannte Verfahren zur Entfernung von Sauerstoff aus Gasströmen durch Inkontaktbringen mit metallischem Kupfer unter Bildung von Kupfer(I)oxid und/oder Kupfer(11)oxid; oder das bekannte Verfahren zur Entfernung von Kohlenmonoxid aus Gasströmen durch Inkontaktbringen mit Kupfer(I)oxid und/oder Kupfer(I)oxid unter Bildung von Kohlendioxid und metallischen Kupfer). Bei einer reinen Adsorption wie auch bei der Katalyse wird das Adsorbat oder sein Reaktionsprodukt durch Desorption wieder von der Oberfläche entfernt, bei der Absorption ist meist eine chemische Regenerierung des Absorbens nötig. Sowohl bei der Katalyse als auch bei der Absorption ist der einleitende Schritt jedenfalls eine Adsorption, und ob ein adsorptives Reinigungsverfahren letztendlich (z. B. bei der Regenerierung der Adsorptionsmasse) in einem katalytischen oder einem absorptiven Schritt mündet oder ein rein adsorptives Verfahren vorliegt, hängt vom Einzelfall ab. Im Rahmen der vorliegenden Erfindung bedeutet "adsorptiv", dass während der Entfernung von CO aus dem zu reinigenden Stoffstrom kein Reaktionsprodukt des Kohlenmonoxids in den Stoffstrom abgegeben wird, und die verwendete Adsorptionsmasse chemisch im wesentlichen unverändert bleibt, also ihre Zusammensetzung nicht oder nur in unwesentlicher Weise ändert. Ob bei der Regenerierung des erfindungsgemäßen Adsorbens dagegen Kohlenmonoxid oder ein Umsetzungsprodukt davon abgegeben werden, also Katalyse stattfindet oder nicht, ist für die Erfindung unerheblich.
Adsorptionsmassen oder Absorptionsmassen werden umgangssprachlich oft auch als "Katalysatoren" bezeichnet, ohne bei ihrem bestimmungsgemäßen Einsatz tatsächlich katalytisch zu wirken.

Die Adsorptionsmasse enthält Kupfer- und Zirkonoxide. Kupfer kann vollständig oder teilweise auch als metallisches Kupfer vorliegen und liegt ansonsten in Form von Cu(I)- und Cu(11)-Oxiden vor. Vorzugsweise liegt Kupfer teilweise metallisch und teilweise in Form von Cu(I)- und Cu(II)-Oxiden vor, d.h. die Adsorptionsmasse ist "anreduziert" (oder "anoxidiert", je nach Betrachtungsweise). Die Adsorptionsmasse enthält im Allgemeinen Kupfer in einer Menge, die als CuO gerechnet mindestens 30 Gew.-%, vorzugsweise mindestens 50 Gew.-% und in besonders bevorzugter Weise mindestens 70 Gew.-%, sowie im Allgemeinen höchstens 99,8 Gew.-%, vorzugsweise höchstens 95 Gew.-% und in besonders bevorzugter Weise höchstens 90 Gew.-% Kupferoxid CuO, jeweils bezogen auf die Gesamtmenge der Adsorptionsmasse entspricht. Die Adsorptionsmasse enthält ferner im Allgemeinen Zirkondioxid ZrO₂ in einer Menge von mindestens 0,2 Gew.-%, vorzugsweise mindestens 5 Gew.-% und in besonders bevorzugter Weise mindestens 10 Gew.-% sowie im Allgemeinen höchstens 70 Gew.-%, vorzugsweise höchstens 50 Gew.-% und in besonders bevorzugter Weise höchstens 30 Gew.-%, jeweils bezogen auf die Gesamtmenge der Adsorptionsmasse.
Die prozentualen Mengen der Komponenten der Adsorptionsmasse addieren sich stets zu 100 Gew.-%.

Die Adsorptionsmasse enthält mit Ausnahme unvermeidlicher Verunreinigungen kein Zinkoxid (ZnO).
Die Adsorptionsmasse besteht vorzugsweise im wesentlichen aus Kupfer(oxid) und Zirkondioxid, das heißt, neben Kupfer(oxid) und Zirkonoxid enthält die Adsorptionsmasse keine weiteren Bestandteile, abgesehen von unwesentlichen Bestandteilen, die beispielsweise noch aus der Fertigung mitgeschleppt werden, wie Überreste von Ausgangsstoffen und Reagenzien, Hilfsstoffe zur Formgebung und Ähnliches.

Eine sehr gut geeignete Adsorptionsmasse besteht im wesentlichen beispielsweise aus ca. 80 Gew.-% CuO und ca. 20 Gew.-% ZrO₂. Eine andere, ebenfalls sehr gut geeignete Adsorptionsmasse besteht in Reinform beispielsweise aus ca. 85 Gew.-% CuO und ca. 15 Gew.-% ZrO₂.

Die Adsorptionsmasse kann, muss aber nicht unbedingt in Reinform vorliegen. Es ist möglich, sie mit Hilfsstoffen zu vermischen oder sie auf einen inerten Träger aufzubringen. Geeignete inerte Träger sind die bekannten Katalysatorträger wie beispielsweise Aluminiumoxid, Siliciumdioxid, Zirkondioxid, Alumosilikate, Tone, Zeolithe, Kieselgur und Ähnliche.

Die Adsorptionsmasse wird hergestellt wie bekannte oxidische Katalysatoren. Ein bequemes und bevorzugtes Verfahren zur Herstellung der Adsorptionsmasse umfasst die folgenden Verfahrensschritte in der genannten Reihenfolge:
a) Herstellen einer Lösung der Komponenten der Adsorptionsmasse und/oder von löslichen Ausgangsverbindungen davon;
b) Fällen eines Festkörpers aus dieser Lösung durch Zugabe einer Base;
c) Abtrennung und Trocknung des Festkörpers;
d) wahlweise eine Kalzination des Festkörpers;
e) Verformung des Festkörpers zu Formkörpern; und
f) wahlweise eine Kalzination der Formkörper;
mit der Maßgabe, dass mindestens einer der beiden Kalzinationsschritte d) oder f) durchgeführt wird.

Im ersten Verfahrensschritt, Schritt a), wird in üblicher Weise eine Lösung der Komponenten der Adsorptionsmasse hergestellt, beispielsweise durch Lösen in einer Säure wie Salpetersäure. Wahlweise werden statt der Komponenten der Adsorptionsmasse auch deren Ausgangsverbindungen verwendet, beispielsweise die Nitrate, Carbonate, Hydroxicarbonate der Metalle in einer wässrigen Lösung, die auch sauer, beispielsweise salpetersauer sein kann, gelöst. Das Mengenverhältnis der Salze in der Lösung wird gemäß der gewünschten Endzusammensetzung der Adsorptionsmasse stöchiometrisch berechnet und eingestellt.

Aus dieser Lösung wird im Schritt b) ein Festkörper als Vorläufer der Adsorptionsmasse gefällt. Dies erfolgt in üblicher Weise, vorzugsweise durch Erhöhung des pH-Werts der Lösung durch Zugabe einer Base, etwa durch Zugabe von Natronlauge oder Sodalösung.

Das entstehende feste Fällprodukt wird vor der Trocknung in Schritt c) in der Regel von der überstehenden Lösung abgetrennt, etwa durch Filtrieren oder Dekantieren, und mit Wasser frei von löslichen Bestandteilen wie Natriumnitrat gewaschen. Das Fällprodukt wird dann normalerweise vor der Weiterverarbeitung mit üblichen Trocknungsmethoden getrocknet. Im allgemeinen genügt dazu eine Behandlung bei leicht erhöhter Temperatur, etwa mindestens 80°C, vorzugsweise mindestens 100°C und in besonders bevorzugter Weise mindestens 120 °C statt, über einen Zeitraum von 10 min bis 12 Stunden, vorzugsweise 20 min bis 6 Stunden und in besonders bevorzugter Weise 30 min bis 2 Stunden. Es ist auch möglich und besonders bequem, das Produkt der Fällung direkt - ein gewisser Alkali-, zum Beispiel Natriumgehalt der Adsorptionsmasse stört im Allgemeinen nicht - oder nach Waschen durch Sprühtrocknung zu einem trockenen weiterverarbeitungsfähigen Pulver umzuwandeln.

Im Anschluss an die Trocknung wird das gefällte und getrocknete Vorprodukt der Adsorptionsmasse wahlweise dem Kalzinationsschritt d) unterzogen. Die angewendete Kalzinationstemperatur liegt dabei im Allgemeinen bei mindestens 200°C, vorzugsweise mindestens 250 °C und in besonders bevorzugter Weise bei mindestens 270°C, sowie im Allgemeinen bei höchstens 500 °C, vorzugsweise höchstens 450°C und in besonders bevorzugter Weise bei höchstens 410°C. Die Kalzinationsdauer beträgt im Allgemeinen mindestens 10 Minuten, vorzugsweise mindestens 20 Minuten und in besonders bevorzugter Weise mindestens 30 Minuten sowie im Allgemeinen höchstens 12 Stunden, vorzugsweise höchstens 6 Stunden und in besonders bevorzugter Weise höchstens 4 Stunden. Der Trocknungsschritt c) und der Kalzinationsschritt d) können direkt ineinander übergehen.
Nach dem Trocknungsschritt c) oder dem Kalzinationsschritt d) wird die Adsorptionsmasse oder ihr Vorläufer im Formgebungsschritt e) mit üblichen Formgebungsverfahren wie Verstrangen, Tablettieren oder Pelletisieren zu Formkörpern wie Stränglingen oder Extrudaten, Tabletten oder - auch kugelförmigen - Pellets verarbeitet.
Nach dem Formgebungsschritt wird die Adsorptionsmasse oder ihr Vorläufer wahlweise einem Kalzinationsschritt f) unterzogen. Die in Schritt f) anzuwendenden Kalzinationsbedingungen sind mit denen des Kalzinationsschritts d) identisch.
Die Adsorptionsmasse wird im Zuge ihrer Herstellung mindestens einem der beiden Kalzinationsschritte d) oder f) unterzogen, wahlweise auch beiden. Bei dem oder den Kalzinationschritten wird der Adsorptionsmassenvorläufer zur eigentlichen Adsorptionsmasse umgewandelt und unter Anderem wie üblich auch die BET-Oberfläche und das Porenvolumen der Adsorptionsmasse eingestellt, wobei bekanntermaßen die BET-Oberfläche und das Porenvolumen mit steigender Kalzinationsdauer und Kalzinationstemperatur sinken.

Vorzugsweise wird zumindest insgesamt so lange kalziniert, dass die BET-Oberfläche der Adsorptionsmasse einen Wert im Bereich von mindestens 40 und höchstens 100 m²/g aufweist. Das Porenvolumen der Adsorptionsmasse, gemessen als Wasseraufnahme, wird bei der Kalzination auf einen Wert von mindestens 0,05 ml/g eingestellt. Diese Werte sind für die Adsorptionsmasse bevorzugt.

Die Adsorptionsmasse kann auch, wie oben erwähnt, auf einem Träger abgeschieden werden. Dies geschieht durch übliche Tränkverfahren oder Auffällverfahren. Ein Auffällverfahren ist bekanntlich ein Fällverfahren in Gegenwart eines Trägers oder eines Trägervorläufers. Zur Durchführung eines Auffällverfahrens wird vorzugsweise im oben ausgeführten Fällverfahren der in Schritt a) hergestellten Lösung ein Träger oder Trägervorläufer zugesetzt. Falls der Träger bereits in Form von vorgeformten fertigen Formkörpern vorliegt, also ein reines Tränkverfahren entfällt der Formgebungsschritt e), ansonsten wird der Träger im Zuge der Verarbeitung des Vorprodukts der Adsorptionsmasse durch Fällung, Trocknung, Kalzinierung und Formgebung mit ausgebildet.
Ein bevorzugtes Tränkverfahren zur Herstellung der erfindungsgemäßen Adsorptionsmasse wird mit vorgeformten Trägern durchgeführt und umfasst die folgenden Verfahrensschritte in der genannten Reihenfolge:
a) Herstellen einer Lösung der Komponenten der Adsorptionsmasse und/oder von löslichen Ausgangsverbindungen davon;
b) Tränken eines vorgeformten Trägers mit dieser Lösung;
c) Trocknung des getränkten Trägers; und
d) Kalzination des getränkten und getrockneten Trägers.

Verfahrensschritt a) dieses Tränkverfahrens wird wie der oben beschriebene Schritt a) des Fällverfahrens durchgeführt. In Schritt b) wird ein vorgeformter Träger mit der Lösung getränkt. Der vorgeformte Träger hat eine dem Einsatzzweck entsprechend gewählte Form, beispielsweise Stränglinge oder Extrudate, Tabletten oder- auch kugelförmige - Pellets. Die Tränkung wird entweder mit überstehender Lösung oder als Tränkung mit der dem Porenvolumen des Trägers entsprechenden Lösungsmenge ("incipient wetness") durchgeführt. Nach der Tränkung wird der getränkte Träger in Schritten c) und d) wie das Fällprodukt beim Fällverfahren getrocknet und kalziniert. Mit einem vorgeformten Träger sind, entfällt dabei der Formgebungsschritt.

Die Adsorptionsmassen-Formkörper werden zu ihrer Verwendung in einen üblicherweise als "Adsorber", gelegentlich auch "Reaktor" bezeichneten Behälter gefüllt, in dem sie mit dem zu reinigenden Stoffstrom in Kontakt gebracht werden.

Die fertige Adsorptionsmasse wird vorzugsweise vor ihrem Einsatz zur Adsorption von CO aktiviert. Es ist auch empfehlenswert, sie vor ihrem Einsatz auch nochmals zu trocknen, um Spuren anhaftender Feuchtigkeit zu entfernen und die Adsorptionskapazität zu erhöhen.

Bequemerweise führt man diese nochmalige Trocknung und die Aktivierung im Adsorber durch, da ansonsten ein hoher Aufwand nötig ist, um die einsatzfertige aktivierte Adsorptionsmasse beim Einfüllen in den Adsorber vor Luft und Feuchtigkeit zu schützen.

Die nochmalige Trocknung des Vorläufers der Adsorptionsmasse, falls erforderlich, wird durch Heizen des Vorläufers auf eine Temperatur von im Allgemeinen mindestens 100 °C, vorzugsweise mindestens 150 °C und in besonders bevorzugter Weise mindestens 180 °C sowie im Allgemeinen höchstens 300 °C, vorzugsweise höchstens 250 °C und in besonders bevorzugter Weise höchstens 220 °C erreicht. Eine geeignete Trocknungstemperatur beträgt beispielsweise ca. 200 °C. Der Vorläufer wird so lange bei der Trocknungstemperatur gehalten, bis nur noch nicht mehr störende Reste anhaftender Feuchtigkeit vorhanden sind; dies ist im Allgemeinen bei einer Trocknungsdauer von mindestens 10 Minuten, vorzugsweise mindestens 30 Minuten und in besonders bevorzugter Weise mindestens 1 Stunde sowie im Allgemeinen höchstens 100 Stunden, vorzugsweise höchstens 10 Stunden und in besonders bevorzugter Weise höchstens 4 Stunden der Fall. Vorzugsweise findet die Trocknung in einem Gasstrom statt, um die Feuchtigkeit aus der Schüttung abzutransportieren. Dazu kann beispielsweise trockene Luft verwendet werden, besonders bevorzugt ist es jedoch, die Schüttung mit einem Inertgas zu durchströmen, geeignet sind hier insbesondere Stickstoff oder Argon.

Die Aktivierung erfolgt durch zumindest teilweise Reduktion des in der Adsorptionsmasse enthaltenen Kupfers zu Kupfermetall oder Kupfer(1)-Verbindungen. Dies kann im Prinzip durch jedes Reduktionsmittel erfolgen, das Kupfer aus den Oxidationsstufen I oder II zur Oxidationsstufe 0 reduzieren kann. Dies kann mit flüssigen oder gelösten Reduktionsmitteln erfolgen, in diesem Fall muss nach der Aktivierung getrocknet werden. Sehr viel bequemer ist deshalb die Reduktion mit einem gasförmigen Reduktionsmittel nach der Trocknung, vor allem die Reduktion mit Wasserstoff durch Überleiten eines Wasserstoff enthaltenen Gases. Die bei der Aktivierung anzuwendende Temperatur beträgt im allgemeinen mindestens 80 °C, vorzugsweise mindestens 100 °C und in besonders bevorzugter Weise mindestens 110 °C sowie im allgemeinen höchstens 200 °C, vorzugsweise höchstens 160 °C und in besonders bevorzugter Weise höchstens 130 °C erreicht. Eine geeignete Aktivierungstemperatur ist beispielsweise ca. 120 °C. Die Reduktion ist exotherm. Die Menge an zugeführtem Reduktionsmittel ist so einzustellen, dass das gewählte Temperaturfenster nicht verlassen wird. Der Verlauf der Aktivierung kann anhand der in der Schüttung des Adsorptionsmittels gemessenen Temperatur verfolgt werden ("temperaturprogrammierte Reduktion, TPR").

Eine bevorzugte Methode zur Aktivierung der Adsorptionsmasse ist es, im Anschluss an eine unter einem Inertgasstrom durchgeführte Trocknung die gewünschte Aktivierungstemperatur einzustellen und dem Inertgastrom eine geringe Menge Wasserstoff beizumischen.

Inertgas ist jedes unter den Bedingungen inerte Gas oder Gasgemisch, beispielsweise Stickstoff, Helium, Neon, Krypton, Xenon, Argon oder ihre Gemische. Vorzugsweise wird Stickstoff verwendet.

Ein zur Aktivierung geeignetes Gasgemisch enthält zu Beginn beispielsweise mindestens 0,1 Vol.-% Wasserstoff in Stickstoff, vorzugsweise mindestens 0,5 Vol.-% und in besonders bevorzugter Weise mindestens 1 Vol.-%, sowie höchstens 10 Vol.-%, vorzugsweise höchstens 8 Vol.-% und in besonders bevorzugter Weise höchstens 5 Vol.-%. Ein geeigneter Wert ist beispielsweise 2 Vol.-%. Diese Anfangskonzentration wird entweder beibehalten oder erhöht, um das gewünschte Temperaturfenster zu erreichen und zu halten.

Die Reduktion ist vollständig, wenn trotz konstantem oder steigendem Pegel des Reduktionsmittels die Temperatur in der Schüttung der Adsorptionsmasse zurückgeht. In bevorzugter Weise wird das in der Adsorptionsmasse enthaltene Kupfer nicht vollständig zu metallischem Kupfer reduziert, so dass die aktivierte Adsorptionsmasse sowohl metallisches wie auch oxidisches Kupfer enthält. Eine typische Aktivierungsdauer für diesen Fall beträgt im allgemeinen mindestens 1 Stunde, vorzugsweise mindestens 10 Stunden und in besonders bevorzugter Weise mindestens 15 Stunden sowie im allgemeinen höchstens 100 Stunden, vorzugsweise höchstens 50 Stunden und in besonders bevorzugter Weise höchstens 30 Stunden.
Sofern der Anteil an metallischem Kupfer zu hoch werden sollte, kann die Adsorptionsmasse in analoger Weise auch oxidiert werden. Dazu wird vorzugsweise statt eines Wasserstoff-/Inertgas-Gemisches ein Sauerstoff-/Inertgas-Gemisch über die Adsorptionsmasse geleitet. Alternativ kann die Aktivierung auch bewusst durch vollständige Reduktion der Adsorptionsmasse mit anschließender Reoxidation auf einen gewünschten Oxidationsgrad erfolgen. Ein geeignetes Verfahren ist beispielsweise die Reduktion bei 200 °C im Wasserstoff- oder Wasserstoff-/Inertgasstrom mit einer anschließenden Reoxidation bei Raumtemperatur mit einem Sauerstoff-/Inertgasstrom. Derartige Verfahren sind für kupferhaltige Katalysatoren bekannt.

Im Anschluss an die Aktivierung ist die Adsorptionsmasse einsatzbereit.
Das erfindungsgemäße adsorptive Verfahren ist ein Verfahren zur Entfernung von Kohlenmonoxid aus Stoffströmen durch Adsorption, das dadurch gekennzeichnet ist, dass man den Kohlenmonoxid enthaltenden Stoffstrom mit einer Adsorptionsmasse in Kontakt bringt, die Kupfer- und Zirkonoxide enthält. Das erfindungsgemäße adsorptive Verfahren ist also durch die Verwendung der beschriebene Adsorptionsmasse gekennzeichnet. Ein Vorzug des erfindungsgemäßen adsorptiven Verfahrens ist seine Anwendbarkeit auf Stoffströme, die entweder sauerstofffrei sind, bei einer Temperatur vorliegen, die für die übliche katalytische Umsetzung von Kohlenmonoxid mit Sauerstoff zu Kohlendioxid nicht ausreicht, oder bei deren weiterer Verwendung Kohlendioxid oder Oxygenate stören.
Im Prinzip kann mit dem erfindungsgemäßen adsorptiven Verfahren jeder Stoffstrom von Verunreinigungen durch Kohlenmonoxid befreit werden, beispielsweise Inertgasströme (Stickstoff, Helium, Neon, Krypton, Xenon und/oder Argon) oder Kohlenwasserstoffströme wie beispielsweise Alkane (Methan, Ethan, Propan, Butan, ihre Gemische, Isomeren und Isomerengemische) oder Alkene (auch "Olefine" genannt) wie Ethen, Propen, 1- Buten, 2-Buten, 1,3-Butadien und/oder Styrol.

Es ist ebenso möglich, die Adsorptionsmasse in nicht-adsorptiver Weise zur Entfernung von Kohlenmonoxid zu verwenden. Dies ist insbesondere vorteilhaft, wenn der von Kohlenmonoxid zu befreiende Stoffstrom neben Kohlenmonoxid auch Sauerstoff enthält, bei einer für die katalytische Umsetzung von Sauerstoff mit Kohlenmonoxid ausreichend hohen Temperatur vorliegt, und bei seiner weiteren Verwendung Kohlendioxid oder Oxygenate nicht stören. So kann Kohlenmonoxid aus Kohlenmonoxid und Sauerstoff enthaltenden Stoffströmen durch katalytische Umsetzung von Kohlenmonoxid mit Sauerstoff an der als Katalysator verwendeten Adsorptionsmasse zu Kohlendioxid umgesetzt und so aus dem Stoffstrom entfernt werden. Ebenso kann Kohlenmonoxid aus Kohlenmonoxid enthaltenden Stoffströmen durch Umsetzung von Kohlenmonoxid mit einer Kupfer(1)- und/oder Kupfer(II)oxid enthaltenden Adsorptionsmasse unter Bildung von metallischem Kupfer zu Kohlendioxid aus dem Stoffstrom entfernt werden. Genauso ist es möglich, Sauerstoff aus Stoffströmen durch Absorption an der metallisches Kupfer enthaltenden Adsorptionsmasse unter Bildung von Kupfer(I)oxid und/oder Kupfer(II)oxid zu entfernen, oder bei Anwesenheit von Wasserstoff durch vom Kupfer katalysierte Bildung von Wasser. Wie mit anderen Kupfer enthaltenden Massen können auch mit der erfindungsgemäßen Adsorptionsmasse nicht nur Kohlenmonoxid, Sauerstoff und mit letzterem auch Wasserstoff, sondern auch sonstige mit Kupfer oder Kupferoxid reagierende Verunreinigungen wie beispielsweise elementares Quecksilber und/oder Quecksilber, Schwefel, Antimon und/oder Arsen enthaltende Verbindungen aus Stoffströmen entfernt werden. Mit anderen Worten: Die Adsorptionsmasse kann in allen bekannten Verfahren eingesetzt werden, in denen Kupfer enthaltende Festkörper katalytisch, absorptiv oder als Reaktionspartner verwendet werden.
Bevorzugterweise wird das erfindungsgemäße adsorptive Verfahren zur Entfernung von Kohlenmonoxid aus Alkenströmen verwendet, insbesondere zur Entfernung von Kohlenmonoxid aus Alkenströmen, die üblicherweise flüssig vorliegen. Flüssig vorliegende Alkene haben typischerweise - abgesehen von der Anwendung unüblich hoher Drücke - nicht die zur katalytischen Entfernung von Kohlenmonoxid durch Umsetzung mit Sauerstoff notwendige Temperatur, zudem würde bei der anschließenden Verwendung zur Polymerisation die Oxygenatbildung stören.
Besonders geeignet ist das erfindungsgemäße adsorptive Verfahren zur Entfernung von Kohlenmonoxid aus Propen, 1-Buten, 2-Buten, 1,3-Butadien, Butengemischen, Buten-/Butadiengemischen oder Styrol, um den Kohlenmonoxidgehalt auf den für "polymer grade"-Olefine zulässige Werte zu senken. In einer ganz besonders bevorzugten Ausführungsform wird mit dem erfindungsgemäßen Verfahren Kohlenmonoxid aus flüssigem Propen adsorptiv entfernt.
Das erfindungsgemäße adsorptive Verfahren ermöglicht die Entfernung von Kohlenmonoxid aus Stoffströmen. Es ist besonders geeignet zur Entfernung von Kohlenmonoxid aus Stoffströmen, die im Allgemeinen mindestens 0,001 ppm (bei Gasen Vol.-ppm, bei Flüssigkeiten Gew.-ppm), vorzugsweise mindestens 0,01 ppm, sowie im Allgemeinen höchstens 1000 ppm, vorzugsweise höchstens 100 ppm und in besonders bevorzugter Weise höchstens 10 ppm Kohlenmonoxid enthalten. Für relativ hohe Anfangskonzentrationen an Kohlenmonoxid ist es meist wirtschaftlicher, vorab ein anderes bekanntes Reinigungsverfahren wie Destillation, katalytische Oxidation des Kohlenmonoxids mit Sauerstoff zu Kohlendioxid oder Oxidation des Kohlenmonoxids mit Kupferoxid unter Bildung von metallischem Kupfer und Kohlendioxid, wahlweise mit nachfolgender Abtrennung von Kohlendioxid und Oxygenaten durchzuführen, da sonst die Adsorptionskapazität der Adsorptionsmasse zu schnell erreicht werden kann.
Zur Durchführung des erfindungsgemäßen adsorptiven Verfahrens wird der von Kohlenmonoxid zu befreiende Stoffstrom im Adsorber über die Schüttung der Adsorptionsmassen-Formkörper geleitet.
Die Temperatur ist für das erfindungsgemäße adsorptive Verfahren aus technischer Sicht nicht oder nur wenig kritisch. Typische Temperaturen liegen im Bereich von mindestens -270 °C, vorzugsweise mindestens -100 °C und in besonders bevorzugter Weise bei -40 °C, sowie höchstens 300 °C, vorzugsweise höchstens 200 °C und in besonders bevorzugter Weise höchstens 100 °C. Bequemerweise wird die Temperatur nicht gesondert beeinflusst, sondern bei der Temperatur gearbeitet, die der zu behandelnde Stoffstrom hat.
Der wesentliche Parameter, mit dem der Abreicherungsgrad bestimmt wird, ist - neben der wie beschrieben bequemerweise nicht besonders beeinflussten Temperatur - die Kontaktzeit zwischen Stoffstrom und Adsorptionsmasse. Diese Kontaktzeit wird durch die Geschwindigkeit des Stoffstroms und das Volumen des Adsorptionsmassen-Betts bestimmt. Meist wird der Volumenstrom des zu reinigenden Stoffstroms durch die Kapazität voran- oder nachgeschalteter Anlagen vorgegeben sein. Weiterhin ist die Adsorptionskapazität der Adsorptionsmasse begrenzt, so dass eine bestimmte Menge Adsorptionsmasse lediglich über einen bestimmten Zeitraum für das erfindungsgemäβe Verfahren benutzt werden kann, bevor sie regeneriert werden muss. Dies macht zwar zunächst die Verwendung einer möglichst großen Menge Adsorptionsmasse wünschenswert, dem stehen allerdings die mit der Adsorbergröße steigenden Kosten entgegen. Die Menge an Adsorptionsmasse im Adsorber wird deshalb im Einzelfall so gewählt, dass einerseits der gewünschte Abreicherungsgrad und andererseits eine tolerierbar kurze Betriebszeit eines Adsorbers zwischen zwei Regenerierungen der Adsorptionsmasse erreicht wird. Vorteilhafterweise werden mindestens zwei Adsorber vorgesehen, von denen mindestens einer mit zu reinigendem Stoffstrom beaufschlagt werden kann, während die Adsorptionsmasse in mindestens einem anderen regeneriert wird. Dies ist eine routinemäßige Optimierungsaufgabe für den Fachmann.

Je nach der gewählten Adsorbergröße wird die maximale Aufnahmekapazität der darin enthaltenen Adsorptionsmasse für Kohlenmonoxid früher oder später erreicht, so dass sie regeneriert werden muss.

Zur Regenerierung der Adsorptionsmasse wird zunächst der zu reinigende Stoffstrom abgestellt, vorzugsweise wird er in einen parallelen, mit frischer oder regenerierter Adsorptionsmasse gefüllten Adsorber geleitet.
Die zu regenerierende Adsorptionsmasse wird anschließend regeneriert. Dies geschieht durch Desorption. Dabei ist es unerheblich, ob vor der Desorption das adsorbierte Kohlenmonoxid katalytisch mit möglicherweise adsorbiertem Sauerstoff oder rein chemisch durch Reaktion mit in der Adsorptionsmasse vorhandenem Kupferoxid zu Kohlendioxid oder auf andere Weise, etwa mit etwaige vorhandenem Wasserstoff zu Methanol oder Methan abreagiert, und diese Reaktionsprodukte anschließend desorbieren, wesentlich ist die Wiederherstellung der Adsorptionskapazität der Adsorptionsmasse.
Die Desorption wird durch Überleiten eines Fluids, vorzugsweise eines Gases, durch Erhöhen der Temperatur oder durch eine Kombination dieser Maßnahmen durchgeführt. In bevorzugter Weise wird der Adsorber mit der zu regenerierenden Adsorptionsmasse mit einem Gas durchströmt und dabei aufgeheizt. Das Gas kann inert sein wie beispielsweise Stickstoff, Methan oder Argon, es ist jedoch auch möglich, Wasserstoff zu verwenden, in diesem Fall wird das CO zu Methanol oder Methan umgesetzt. Die Desorptionstemperatur wird im Allgemeinen auf einen Wert von mindestens 50 °C, vorzugsweise mindestens 100 °C und in besonders bevorzugter Weise mindestens 150 °C sowie im Allgemeinen höchstens 500 °C, vorzugsweise höchstens 450 °C und in besonders bevorzugter Weise höchstens 400 °C eingestellt. Beispielsweise ist eine Desorptionstemperatur von ca. 300 °C geeignet. Die Dauer der Regenerierung ist typischerweise im Allgemeinen mindestens 1 Stunde, vorzugsweise mindestens 10 Stunden und in besonders bevorzugter Weise mindestens 15 Stunden sowie im Allgemeinen höchstens 100 Stunden, vorzugsweise höchstens 50 Stunden und in besonders bevorzugter Weise höchstens 30 Stunden.
Um aus dem Kupfer verlorenen Sauerstoff zu ersetzen, ist es oft vorteilhaft, die Desorption mit einem Inertgas durchzuführen - bevorzugt sind Stickstoff oder Argon - , das in Spuren Sauerstoff enthält. Bequemerweise wird zur Desorption Stickstoff verwendet, der im Allgemeinen Sauerstoff in einer Menge von mindestens 1 ppm, vorzugsweise mindestens 5 ppm und in besonders bevorzugter Weise mindestens 10 ppm sowie im Allgemeinen höchstens 300 ppm, vorzugsweise höchstens 250 ppm und in besonders bevorzugter Weise höchstens 200 ppm enthält.

Die eigentliche Desorption kann auch mit dem Entfernen restlichen zu reinigenden Stoffstroms aus dem Adsorber durch Spülen des Adsorbers, zweckmäßigerweise mit dem zur Desorption verwendeten Gasstrom bei Normaltemperatur eingeleitet werden. Im Anschluss an diese Regenerierung ist die Adsorptionsmasse im Allgemeinen sofort zum erneuten Einsatz bereit. Im Einzelfall - insbesondere wenn sich der gewünschte Reduktionsgrad zu stark verändert hat - kann es empfehlenswert oder erforderlich sein, die Adsorptionsmasse einer erneuten Einstellung des Reduktionsgrads zu unterziehen.

Mit der Adsorptionsmasse und dem erfindungsgemäßen adsorptiven Verfahren ist es möglich, Kohlenmonoxid aus Stoffströmen einfach und in wirtschaftlicher Weise zu entfernen. Die so gereinigten Stoffströme können anschließend bestimmungsgemäß verwendet werden.

### Beispiele

### Allgemeine Vorschrift zur Herstellung der Adsorptionsmassen

In einem 10 I-Reaktionsgefäß werden 1200 ml dest. Wasser vorgelegt und auf 70 °C erwärmt. Nach Erreichen dieser Temperatur werden unter Rühren Lösungen der Metallnitrate in der zum Erreichen der gewünschten Zusammensetzung notwendigen Menge zugepumpt. Durch gleichzeitige Zudosierung einer 20 Gew.-%igen Natriumcarbonatlösung wird der pH-Wert der Mischung auf 6,5 gehalten. Die entstehende Suspension wird 120 Minuten bei 70 °C und pH 6,5 weitergerührt. Anschließend wird die heiße Suspension filtriert und der Feststoff mit kalten dest. Wasser nitratfrei gewaschen. Das Pulver wird getrocknet, 4 Stunden bei 300 °C kalziniert und zu 3 x 3 mm-Tabletten tablettiert. Nach nochmaliger Trocknung und vollständiger Reduktion mit einem Wasserstoff-/Stickstoff-Gemisch wird die Adsorptionsmasse durch Inkontaktbringen mit einem Gemisch von 0,6 Vol.-% Sauerstoff in Stickstoff über eine Stunde bei Raumtemperatur anoxidiert.

Nach dieser allgemeinen Vorschrift wurden die in der Tabelle angegebenen Adsorptionsmassen erhalten.

### Allgemeine Vorschrift zur Bestimmung der CO-Adsorptionskapazität

85 ml der Adsorptionsmasse werden bei Raumtemperatur und Umgebungsdruck (d.h., nur der zur Durchströmung der Schüttung notwendige Druck wird vor Reaktor angelegt) in einem Rohrreaktor (Adsorber) mit einem Gasgemisch aus 100 ppm CO in Propylen beaufschlagt, mit einer GHSV von 1765 h⁻¹ (entsprechend 150 NI Propylen/h). Die CO-Konzentration im Abgas wird kontinuierlich gemessen. Angegeben werden die 10%-, 20%- und 50%-" Durchbruchspunkte", d.h. jeweils das Verhältnis des der Adsorptionsmasse zugeführten Volumens an CO (als Reinstoff berechnet) pro Volumen der Adsorptionsmasse, bei dem 10 %, 20 % oder 50 % der CO-Konzentration des dem Adsorber zugeführten Gasgemisches nach Durchtritt durch den Adsorber gemessen werden. Diese Durchbruchspunkte sind demnach ein Maß für die Adsorptionskapazität der Adsorptionsmasse.

Nach dieser allgemeinen Vorschrift wurden die in der Tabelle angegebenen Durchbruchspunkte gemessen

| Beispiel Nr. | Zusammensetzung [Gew.-%] | | | Durchbruchspunkte [I CO /I Adsorptionsmasse] | | |
|---|---|---|---|---|---|---|
| | CuO | ZrO₂ | ZnO | 10 | 20 | 50 |
| 1 (Vergleich) | 50 | 10 | 40 | 1,73 | 2,14 | n.b. |
| 2 (Vergleich) | 60 | 10 | 30 | 2,14 | 2,61 | 6,02 |
| 3 (Vergleich) | 60 | 20 | 20 | 1,6 | 2,19 | 4,18 |
| 4 (Vergleich) | 70 | 10 | 20 | 2,56 | 3,19 | 7,16 |
| 5 (Vergleich) | 70 | 20 | 10 | 1,52 | 2,12 | 6,32 |
| 6 (Vergleich) | 80 | 0 | 20 | 1,35 | 1,73 | 3,61 |
| 7 (Vergleich) | 80 | 10 | 10 | 2,62 | 3,28 | n.b. |
| 8 (Vergleich) | 100 | 0 | 0 | 0,05 | 0,06 | 0,1 |
| 9 | 62 | 38 | 0 | 3,91 | n.b. | n.b. |
| 10 | 75 | 25 | 0 | 4,82 | 5,63 | 8,06 |
| 11 | 80 | 20 | 0 | 4,71 | 5,36 | 6,74 |
| 12 | 82 | 18 | 0 | 5,07 | 6,39 | 10,5 |
| 13 | 85 | 15 | 0 | 5,96 | 7,49 | >9,95 |
| 14 | 87 | 13 | 0 | n.b. | n.b. | n.b. |
| 15 | 90 | 10 | 0 | 4,39 | 6,39 | 12,1 |
| 16 | 95 | 5 | 0 | 1,79 | 2,67 | n.b. |

Die Beispiele und Vergleichsbeispiele zeigen, dass die Aufnahmekapazität der Adsorptionsmasse in einem Verfahren zur Entfernung von Kohlenmonoxid aus einem Kohlenmonoxid enthaltenden flüssigen Propylenstrom höher ist als die einer bekannten, jedoch auch Zinkoxid enthaltenden Adsorptionsmasse.

## Patentansprüche

1. Verfahren zur Entfernung von Kohlenmonoxid aus einem Kohlenmonoxid enthaltenden flüssigen Propylenstrom durch Adsorption an einer Adsorptionsmasse, **dadurch gekennzeichnet, dass** man den Kohlenmonoxid enthaltenden flüssigen Propylenstrom mit einer Adsorptionsmasse in Kontakt bringt, die 30 bis 99,8 Gew.-% Kupferoxid und 0,2 bis 70 Gew.-% Zirkonoxid, bezogen auf die Gesamtmenge der Adsorptionsmasse, jedoch kein Zinkoxid enthält.

2. Verfahren nach Anspruch 1, **dadurch gekennzeichnet, dass** die Adsorptionsmasse 50 bis 95 Gew.-% Kupferoxid und 5 bis 50 Gew.-% Zirkonoxid enthält.

3. Verfahren nach Anspruch 1, **dadurch gekennzeichnet, dass** die Adsorptionsmasse 70 bis 90 Gew.-% Kupferoxid und 10 bis 30 Gew.-% Zirkonoxid enthält.

4. Verfahren nach einem der Ansprüche 1, 2 oder 3, **dadurch gekennzeichnet, dass** die Adsorptionsmasse auf einem inerten Träger aufgebracht ist.

## Claims

1. A method of removing carbon monoxide from a liquid propylene stream comprising carbon monoxide by adsorption on an adsorption composition, wherein the liquid propylene stream comprising carbon monoxide is brought into contact with an adsorption composition which comprises from 30 to 99.8% by weight of copper oxide and from 0.2 to 70% by weight of zirconium oxide, based on the total amount of the adsorption composition, but no zinc oxide.

2. The method according to claim 1, wherein the adsorption composition comprises from 50 to 95% by weight of copper oxide and from 5 to 50% by weight of zirconium oxide.

3. The method according to claim 1, wherein the adsorption composition comprises from 70 to 90% by weight of copper oxide and from 10 to 30% by weight of zirconium oxide.

4. The method according to any of claims 1, 2 and 3, wherein the adsorption composition is applied to an inert support.

## Revendications

1. Procédé pour l'élimination de monoxyde de carbone à partir d'un courant de propylène liquide contenant du monoxyde de carbone, **caractérisé en ce qu'**on met le courant de propylène liquide contenant du monoxyde de carbone en contact avec une matière adsorbante qui contient 30 à 99,8 % en poids d'oxyde de cuivre et 0,2 à 70 % en poids d'oxyde de zirconium, par rapport à la quantité totale de la matière adsorbante, mais ne contient pas d'oxyde de zinc.

2. Procédé selon la revendication 1, **caractérisé en ce que** la matière adsorbante contient 50 à 95 % en poids d'oxyde de cuivre et 5 à 50 % en poids d'oxyde de zirconium.

3. Procédé selon la revendication 1, **caractérisé en ce que** la matière adsorbante contient 70 à 90 % en poids d'oxyde de cuivre et 10 à 30 % en poids d'oxyde de zirconium.

4. Procédé selon l'une quelconque des revendications 1, 2 et 3, **caractérisé en ce que** la matière adsorbante est appliquée sur un support inerte.
